# EUROPEAN PATENT APPLICATION

(11) **EP 2 561 877 A1**
(43) Date of publication of application: **27.02.2013**
(21) Application number: 12174951.9
(22) Date of filing: 19.12.2007
(51) Int. Cl.: A61K 31/663, A61K 31/593, A61K 9/20, A61K 9/48, A61P 19/08

(54) **A Composition containing a Bisphosphonic Acid in combination with Vitamin D**

(30) Priority: 20.12.2006 CA 2571538; 20.12.2006 CA 2571398
(62) Divisional of application: 07855595.0
(71) Applicant: Mylan Pharmaceuticals ULC, Calgary AB T2P 3Y7 (CA)
(72) Inventor: Akbarieh, Mostafa, Richmond Hill, Ontario L4E 0C8 (CA); Mohideen, Fazal M., Pickering, Ontario L1V 7A7 (CA); Shah, Shetal, Toronto, Ontario M5A 4T4 (CA)
(74) Representative: Johnson, Stephen William

(57) **Abstract**

The present invention relates to pharmaceutical compositions containing a bisphosphonic acid in combination with a non-activated metabolize of vitamin D for oral administration. The compositions of the invention either do not contain or contain only low concentrations of a glidant. Also provided are methods for preparing such compositions and methods of use thereof, for preventing or treating abnormal bone resorption in mammals.

## Description

### CROSS REFERENCE TO RELATED APPLICATION

This application claims the benefit of co-pending Canadian patent application No. 2,571,538 filed December 19, 2006 and Canadian patent application No. 2,571,398 filed December 20, 2006, both of which are incorporated herein in their entirety.

### FIELD OF THE INVENTION

The present invention pertains to the field of pharmaceutical compositions and methods of preparation thereof and, more particularly, to pharmaceutical compositions comprising a bisphosphonate and a vitamin D compound, and to method of preparation thereof.

### BACKGROUND OF THE INVENTION

Bisphosphonic acids are important in the treatment of bone diseases as well as problems with calcium metabolism such as osteoporosis, Paget's disease, and hypercalcaemia. Bisphosponates, i.e. bisphosphonic acids or soluble, pharmaceutically acceptable salts thereof, are synthetic analogs of the naturally occurring pyrophosphate. Due to their marked affinity for solid-phase calcium phosphate, bisphosphonic acids bind strongly to bone mineral. Pharmacologically active bisphosphonic acids are well known in the art and are potent inhibitors of bone resorption and are therefore useful in the treatment and prevention of diseases involving abnormal bone resorption. Bones serve as support structures but are also involved in various body stimuli signaling and response mechanisms. Therefore simple prosthetics to support the damaged bone do not give a patient the proper active effect that repairing the bone itself would do.

Bisphosphonic acids as pharmaceutical agents are described, for example, in U.S. Patent Nos. 4,687,767, 4,666,895, 4,927,814, 4,942,157, and 4,777,163. Pharmaceutical forms of marketed bisphosphonic acids are oral composition (tablets or capsules) or solutions for intravenous injection or infusion. Many bisphosphonic acids, including alendronic acid, sodium ibandronate, risedronate, pamidronate and clodronate, are currently under clinical development or marketed for the treatment of osteoporosis and metastatic bone diseases (Muhlbauer R.C., Bauss R., Schenk R., Janner M., Bosies E., Strein K., and Fleisch H. BM21.0955 A Potent New Bisphosphonic acid to Inhibit Bone Resorption. J. Bone Miner. Res. 6:1003-1011 (1991)).

Vitamin D3 (Cholecalciferol) is a non-activated form of Vitamin D. It is a precursor of the hydroxylated, biologically active metabolites and analogues of Vitamin D3. Generally cholecalciferol may be activated by hydroxylation into 25-hydroxy-cholecalceiferol, and 25-hydroxy-cholecalciferol may be further hydroxylated at the 1-alpha-position to 1,25-dihydroxy-cholecalciferol (an active form of Vitamin D3). Active forms of Vitamin D can not be given in large dosages due to their toxicity to mammals, whereas the inactive Vitamin D3 form may be given in higher dosages. Vitamin D insufficiency is recognized as causes of metabolic bone disease in adults, characterized by the impairment of calcium and phosphate absorption. Vitamin D deficiency is also characterized by impaired bone mineralization. Sustained vitamin D insufficiency and deficiency are thought to be an important cause of gradual bone loss.

Vitamin D insufficiency can be age related, or due to geographical and seasonal causes. While exposure to sunlight provides most of the vitamin D required for children and young adults, the body can deplete its stored vitamin D because of lack of exposure to sunlight combined with dietary deficiency. However, vitamin D supplementation in food goods fails to ensure adequate intake at times, especially among the elderly who do not frequently consume these foods or where intestinal absorption of calcium is less efficient or where low sunlight exposure is common (thus depletion occurs rapidly).

U.S. Patent No. 4,230,700 issued Oct. 28,1980 discloses the conjoint administration of certain polyphosphonate compounds, in particular bisphosphonic acids and vitamin D-like anti-rachitic compounds for inhibition of the anomalous mobilization of calcium phosphates in animal tissue. U.S. patent 4,330,537 issued May 18, 1982 claims the compositions used in the methods of U.S. Patent 4,230,700. In subjects undergoing bisphosphonic acid therapy, and in particular those subjects with inadequate dietary calcium intake or inadequate calcium absorption, there is a need for supplemental Vitamin D nutrition to facilitate bone formation and mineralization. A single product or preparation comprised of metabolites of Vitamin D and a bisphosphonic acid address this need by ensuring that patients receiving bisphosphonic acid also receive sufficient vitamin D.

Pharmaceutical preparations can be problematic because sometimes they are associated with compression and flowability issues during processing. The increase stickiness and resulting hardness of the granules inhibit the processing, resulting in a retarded dissolution profile and friability problems when excipients are manipulated to improve lubrication or flowability. Thus pharmaceutical preparations have been known to require a "glidant" such as, but not limited to, colloidal silicon dioxide, talc and the like, which improve the flow characteristics of a powder mixture during processing as in patent application WO2005/117906A1. Normally, the glidant makes up between 0.5 and 5.0% of the composition.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

The present invention provides a composition containing a bisphosphonic acid in combination with a vitamin D compound.

In particular, one aspect of the invention provides a pharmaceutical composition comprising: (a) an active pharmaceutical ingredient (API), pharmaceutically acceptable salts thereof, derivatives or hydrates of the API, or mixtures thereof; (b) a vitamin D compound; and (c) one or more excipients selected from fillers, diluents, binders, lubricants or disintegrants, wherein said composition does not include a glidant.

Another aspect of the invention provides a pharmaceutical composition comprising: (a) an active pharmaceutical ingredient (API), pharmaceutically acceptable salts thereof, derivatives or hydrates of the API, or mixtures thereof; (b) a vitamin D compound; (c) lactose; (d) microcrystalline cellulose; and (e) one or more excipients selected from fillers, diluents, binders, lubricants, disintegrants or glidants, wherein the glidant is present at a concentration of from 0 to 0.5% w/w

According to a specific embodiment of the invention the API is a bisphosphonic acid or a pharmaceutically acceptable salt thereof. In a particular embodiment, the bisphosphonic acid is Alendronic acid or a pharmaceutical acceptable salt thereof, for example, alendronate monosodium, alendronate monosodium monohydrate or alendronate monosodium trihydrate.

Further, according to a preferred embodiment of the invention the Vitamin D compound is cholecalciferol. The cholecalciferol in the composition is preferably in an amount of from about 2,800 IU to about 5600 IU while the API or pharmaceutically acceptable salt thereof is in a preferred amount of about 70 mg. In a particularly preferred embodiment, the cholecalciferol in the composition is in an amount of about 2,800 IU or about 5600 IU.

A particularly preferred embodiment of the invention provides a composition as described above, which comprises Alendronic acid as the bisphosphonic acid, Cholecalciferol as the Vitamin D compound, microcyrstalline cellulose (Avicel 302 grade) and an anhydrous form of Spray Dried Lactose. The flow improving excipient such as a glidant, most preferably colloidal silicon dioxide, is either not required or is required at low concentration (i.e., no more than 0.5% by weight).

An advantage of such compositions is that the stickiness typically associated with bisphosphonic acids, in general, seems to have been surprisingly overcome simultaneously along with the improved flowability when the microcyrstalline cellulose (e.g., Avicel 302) and the Lactose Anhydrous (e.g., Spray Dried) are used. Such compositions allow for hardness or dissolution time (DT) levels of the preparation to be sufficient low to reduce or eliminate friability issues, and prevent the composition from crumbling away or not ejecting properly during processing.

Another advantage is that fewer excipients are required to improve flowability of the composition, more specifically the need for a glidant is either removed entirely, or reduced significantly.

Overall, there is the advantage that the compositions herein do not show a retarded dissolution profile in comparison to compositions containing a glidant in the standard amount (typically from greater than 0.5% to 5.0% by weight), as would be expected with such a drastic change in composition even with the complete absence or reduced concentration of glidant.

Another aspect of the present invention provides a process for preparing a pharmaceutical composition as described above, which method comprises the steps of: (a) blending the active pharmaceutical ingredient (API), pharmaceutically acceptable salts thereof, derivatives or hydrates of the API, or mixtures thereof with a vitamin D compound; and (b) adding one or more excipients selected from the group comprising fillers, diluents, binders, lubricants, disintegrants and glidant, wherein said glidant is added so that the final concentration of glidant is from 0 to 0.5% w/w in the final pharmaceutical composition.

According to a further embodiment of the invention the pharmaceutical compositions herein are formulated into solid oral pharmaceuticals or dosage forms, such as, tablets or capsules.

Another aspect of the present invention provides a method for treating a disorder associated with increased bone resorption comprising administering to a patient using a pharmaceutical composition comprising: (a) a bisphosphonic acid, a pharmaceutically acceptable derivative thereof, or mixtures thereof; (b) a vitamin D compound; and (c) one or more excipients selected from fillers, diluents, binders, lubricants, disintegrants or glidants, wherein said glidant is present at a concentration of from 0 to 0.5% w/w. Bone resorption disorders include, but are not limited to osteoporosis, hypercalcaemia, tumour osteolysis and or Paget's disease. The composition for use in this method optionally comprises lactose and microcrystalline cellulose.

### BRIEF DESCRIPTION OF THE FIGURES

Figure 1 depicts the results of testing the dissolution time of tablets prepared without glidant, according to one embodiment of the present invention.
Figure 2 depicts the results of testing the dissolution time of tablets prepared with a low concentration of glidant, according to one embodiment of the present invention.

### DETAILED DESCRIPTION OF THE INVENTION

The present invention relates to the preparation of pharmaceutical compositions containing an active pharmaceutical ingredient (API), or a pharmaceutically acceptable derivative thereof, including salts, esters and solvates thereof, which composition does not include a glidant or includes only a low concentration (i.e., no more than 0.5% by weight) of glidant, but that retains friability, flowability, hardness and DT levels sufficient permit further processing, for example into an oral dosage form. The composition does not crumble away and is ejected properly during processing.

Surprisingly the current inventors have found that high concentrations of excipients are not required in order to improve or allow for an increased flow characteristic of the mixture used in the preparation of the pharmaceutical compositions of the present invention. By utilizing an improved composition, the requirement of a "flow improving" specific excipient, such as a glidant, is no longer necessary or can be included at a low concentration. The combination of alternative excipients, such as, a dense grade of Microcrystalline Cellulose (MCC) and a Spray Dried Lactose anhydrous have surprisingly shown an improved flow of the mixture and resulting in no compression problems during processing.

As used herein, the term "active pharmaceutical ingredient" (API) means a therapeutic compound. When API granules are formulated into tablets, such tablets can also contain coloring agents, lubricants and the like. The granules of the invention can be formulated in a variety of forms such as a tablet, capsule, suspension, reconstitutable powder and suppository.

The therapeutic compound(s) contained within a composition containing the granules of said invention can be formulated as its pharmaceutically acceptable salts thereof. As used herein, "pharmaceutically acceptable salts thereof" refer to derivatives of the disclosed compounds wherein the parent pharmacologically active compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, furnaic, toluenesulfonic, methanesulfonic, ethane disulfonic, oxalic, isethionic, and the like.

The pharmaceutically acceptable salts of the API used in the compositions of the present invention can be synthesized from the parent API which contains a basic or acidic moiety by conventional chemical methods. Generally, such salts can be prepared by reacting the free acid or base forms of these compounds with a predetermined amount of the appropriate base or acid in water or in an organic solvent, or in a mixture of the two. Lists of suitable salts are found in Remington's Pharmaceutical Sciences, 17th ed., Mack Publishing Company, Easton, Pa., 1985, the disclosure of which is hereby incorporated by reference.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The API used in the formulation of the present invention can be a bisphosphonic acid, or a derivative thereof, such as, but not limited to, Etidronate, Clodronate, Tiludronate, Pamidronate, Neridronate, Olpadronate, Alendronate, Ibandronate, Risedronate, Zoledronate or a pharmaceutically acceptable salt, ester or solvate thereof. Such compositions are useful for the treatment of disorders characterized by pathologically increased boned resorption, especially for the treatment of osteoporosis.

According to a particular embodiment of the present invention, the API is [4-Amino-1-hydroxybutylidene]-bisphosphonic acid (alendronic acid).

More specifically, the present invention includes compositions containing a combination of a bisphosphonic acid with a Vitamin D compound. The term "vitamin D" or "vitamin D compound" refers to a group of fat-soluble prohormones, the two major forms of which are vitamin D₂ (or ergocalciferol) and vitamin D₃ (or cholecalciferol). The term "vitamin D" or "vitamin D compound" as used herein also refers to metabolites and other analogues of these substances.

Disintegrants that can be included in the composition of the invention comprise starches such as com starch, potato starch, pregelatinized and modified starches thereof, sweeteners, clays, such as bentonite, microcrystalline cellulose, alginates, sodium starch glycolate, gums such as agar, guar, locust bean, karaya, pecitin and tragacanth. Disintegrants can comprise up to about 20 weight percent and preferably between about 2 and about 10 percent of the total weight of the composition. The microcrystalline cellulose used in the compositions of the present invention is preferably in a dense form. For example, the microcrystalline cellulose used has a density of no less than 0.4 g/ml, such as 0.45 g/ml.

Examples of fillers that can be incorporated in the composition of the present invention include calcium carbonate, calcium phosphate, calcium sulphate, carboxymethylcellulose calcium, carboxymethylcellulose sodium, compressible sugar, confectioner's sugar, dextrates, dextrin, dextrose, dibasic calcium phosphate dihydrate, dibasic calcium phosphate, fructose, glyceryl palmitostearate, glycine, hydrogenated vegetable oil-type 1, kaolin, lactose, maize starch, magnesium carbonate, magnesium oxide, maltodextrin, mannitol, microcrystalline cellulose, polymethacrylates, potassium chloride, powdered cellulose, pregelatinised starch, sodium chloride, sorbitol, starch, sucrose, sugar spheres, talc, tribasic calcium phosphate, and xylitol. When lactose is included in the composition, it is preferably a spray dried lactose, an anhydrous lactose or a spray dried anhydrous lactose. Preferably the lactose has a density of no more than 0.5 g/ml. Specifically, the lactose can be a spray dried anhydrous lactose having a density of 0.67 g/ml.

The composition of the present invention can also include a lubricant. "Lubricant," as used herein, means a material which can reduce the friction arising at the interface of the tablet and the die wall during compression and ejection thereof. Lubricants may also serve to prevent sticking to the punch and, to a lesser extent, the die wall as well. The term "antiadherents" is sometimes used to refer specifically to substances which function during ejection. As used in the present disclosure, however, the term "lubricant" is used generically and includes "antiadherents." Tablet sticking during formation and/or ejection may pose serious production problems such as reduced efficiency, irregularly formed tablets, and non-uniform distribution of intended agents or ingredients to be delivered thereby. These problems are particularly severe with high speed tableting approaches and methods.

Lubricants may be intrinsic or extrinsic. A lubricant which is directly applied to the tableting tool surface in the form of a film, as by spraying onto the die cavity and/or punch surfaces, is known as an extrinsic lubricant. Although extrinsic lubricants can provide effective lubrication, their use requires complex application equipment and methods which add cost and reduce productivity.

Intrinsic lubricants are incorporated in the material to be tableted. Magnesium, calcium and zinc salts of stearic acid have long been regarded as the most efficient intrinsic lubricants in common use. Concentrations of two percent or less are usually effective.

Other traditional intrinsic lubricants include hydrogenated and partially hydrogenated vegetable oils, animal fats, polyethyleneglycol, polyoxyethylene monostearate, talc, light mineral oils, sodium benzoate, sodium lauryl sulphate, magnesium oxide and the like. See U.S. Pat. No. 3,042,531.

Lubricants, according to the present invention, can be used in an amount of up to 1.5 weight percent and preferably between about 0.25 and about 1.0 weight percent of the total composition.

Intrinsic lubricants pose certain serious difficulties when used in conventional tablets. Many lubricants materially retard the disintegration of tablets.

Coloring agents that can be included in the composition of the present invention include titanium dioxide, and dyes suitable for food such as those known as F.D. & C. dyes and natural coloring agents such as grape skin extract, beet red powder, beta-carotene, annato, carmine, turmeric, paprika, etc. The amount of coloring used can range from about 0.1 to about 3.5 weight percent of the total composition.

Flavors incorporated in the composition may be chosen from synthetic flavor oils and flavoring aromatics and/or natural oils, extracts from plants, leaves, flowers, fruits and so forth and combinations thereof. These may include cinnamon oil, oil of wintergreen, peppermint oils, clove oil, bay oil, anise oil, eucalyptus, thyme oil, cedar leave oil, oil of nutmeg, oil of sage, oil of bitter almonds and cassia oil. Also useful as flavors are vanilla, citrus oil, including lemon, orange, grape, lime and grapefruit, and fruit essences, including apple pear, peach, strawberry, raspberry, cherry, plum, pineapple, apricot and so forth. Flavors which have been found to be particularly useful include commercially available orange, grape, cherry and bubble gum flavors and mixtures thereof. The amount of flavoring may depend on a number of factors, including the organoleptic effect desired. Flavors may be present in an amount ranging from about 0.5 to about 3.0 by weight based upon the weight of the composition. Particularly preferred flavors are the grape and cherry flavors and citrus flavors such as orange.

The pharmaceutical composition of the present invention can be processed into a unit dosage form (e.g., tablet, capsule or sachet) and then packaged for distribution. The processing step will vary depending upon the particular unit dosage form. For example, a tablet is generally compressed under pressure into a desired shape and a capsule or sachet employs a simple fill operation. Those skilled in the art are well aware of the procedures used for manufacturing the various unit dosage forms.

Materials incorporated in the pharmaceutical composition of the present invention can be pretreated to form granules that readily lend themselves to tableting. This process is known as granulation. As commonly defined, "granulation" is any process of size enlargement whereby small particles are gathered together into larger, permanent aggregates to yield a free-flowing composition having a consistency suitable for tableting. Such granulated compositions may have consistency similar to that of dry sand. Granulation may be accomplished by agitation in mixing equipment or by compaction, extrusion or globulation.

Tablets according to this aspect of the present invention can be manufactured by well-known tableting procedures. In common tableting processes, material which is to be tableted is deposited into a cavity, and one or more punch members are then advanced into the cavity and brought into intimate contact with the material to be pressed, whereupon compressive force is applied. The material is thus forced into conformity with the shape of the punches and the cavity. Various tableting methods are well known to those skilled in the art and not detailed herein.

When a pharmaceutical composition of the present invention is processed to form a tablet, the tablet's size and shape can be adapted for direct oral administration to a patient, such as a human patient.

The amount of therapeutic compound incorporated in each tablet may be selected according to known principles of pharmacy. An effective amount of therapeutic compound is specifically contemplated. By the term effective amount, it is understood that, with respect to for example pharmaceuticals, a pharmaceutically effective amount is contemplated. A pharmaceutically effective amount is the amount or quantity of a drug or pharmaceutically active substance which is sufficient to elicit the required or desired therapeutic response, or in other words, the amount which is sufficient to elicit an appreciable biological response when administered to a patient.

The following example is given for the purpose of illustrating the present invention and should not be considered as limiting the scope or spirit of the invention in any way.

### EXAMPLES

### Example 1: Composition containing no glidant

The composition described below was characterized in that the combination of Spray Dried Lactose Anydrous together with a denser Microcrystalline Cellulose (Avicel 302) provided sufficient flowability such that, surprisingly, a glidant was no longer required.

| **Composition (without glidant)** | | | |
|---|---|---|---|
| **Description** | **Amt Req'd in Grams** | **Quantity per Unit** | |
| | | **mg** | **%** |
| Alendronate Sodium Trihydrate | 91.37 | 91.37 | 28.55 |
| Lactose Anydrous Spray Dried | 99.98 | 99.98 | 31.24 |
| Plasdone | 12.80 | 12.80 | 4.00 |
| Crosearmellose Sodium | 13.20 | 13.20 | 4.13 |
| Microcrystalline Cellulose (NF PH 302) | 74.90 | 74.90 | 23.41 |
| Cholecalciferol Vitamin D | 26.00 | 26.00 | 8.13 |
| Magnesium Stearate | 1.75 | 1.75 | 0.55 |
| **Total Tablet Weight (Core)** | 320.00 | 320.00 | 100.00 |

The process used to prepare the tablet described above was as follows:
A Prescreen Alendronate Sodium Trihydrate, Lactose Anhydrous NF, Plasdone S630, and Croscarmellose Sodium NF through a #20 mesh and blend for 10 minutes.
B Prescreen Microcrystalline Cellulose PH302 through a #30 mesh and blend for 10 minutes.
C Prescreen Magnesium Stearate Non Bovine through a #60 mesh and blend for 3 minutes.
D Tablet at 15-18Kp.

The dissolution times of resulting tablets were tested and compared to dissolution times for tablets of a commercially available alendronate/Vitamin D tablet (Fosamax D). Dissolution was tested in water using the procedure as set out in USP 23 third supplement.

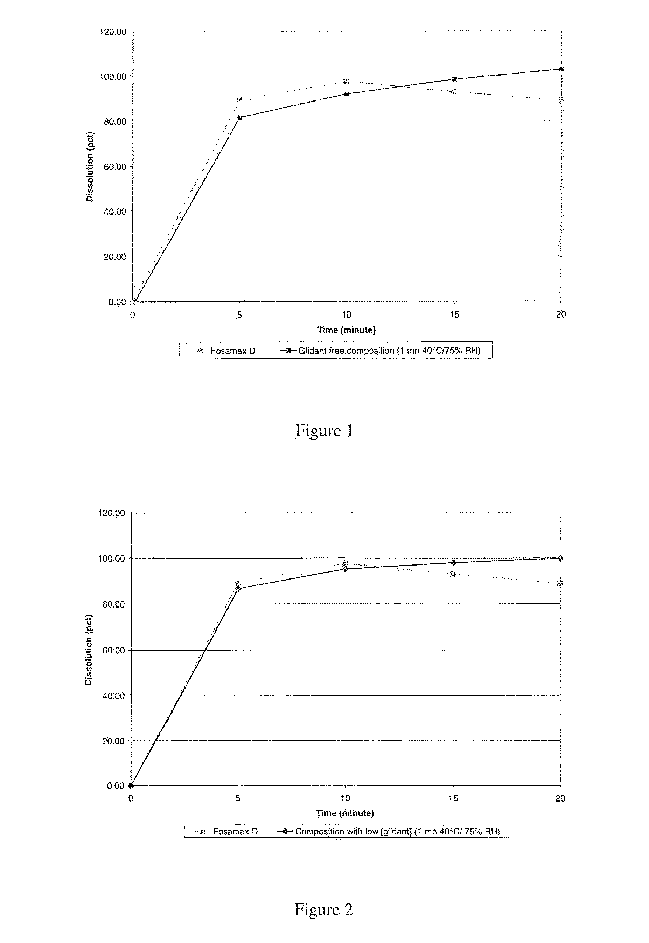

The results depicted in Figure 1 demonstrate that there was essentially no difference between the dissolution profiles of the tablets of the present invention and of the commercially available tablets. Further, the dissolution profiles were compared as prescribed in the Guidance for Industry: Immediate Release Solid Oral Dosage Forms, Scale-Up and Postapproval Changes: Chemistry, Manufacturing, and Controls, In Vitro Dissolution Testing, and In Vivo Bioequivalence Documentation, published by the U.S. Center for Drug Evaluation and Research (CDER), under the heading "*In vitro* Dissolution". In the present study, the similarity factor, f₂ was calculated to be 54.9. Dissolution profiles are recognized to be similar if 50<f₂<100.

### Example 2: Composition comprising low concentration of glidant

The composition described below was characterized in that the combination of a densely Spray Dried Lactose Anydrous together with a dense Microcrystalline Cellulose (Avicel 302) provided an unexpected increase in flowability.

| **Description** | **Amt Req'd in Grams** | **Quantity per Unit** | |
|---|---|---|---|
| | | **mg** | **%** |
| Alendronate Sodium Trihydrate | 91.37 | 91.37 | 28.55 |
| Lactose Anhydrous Spray Dried | 98.38 | 98.38 | 30.74 |
| Plasdone | 12.80 | 12.80 | 4.00 |
| Croscarmellose Sodium | 13.20 | 13.20 | 4.13 |
| Microcrystalline Cellulose (NF PH 302) | 74.90 | 74.90 | 23.41 |
| Cholecalciferol Vitamin D | 26.00 | 26.00 | 8.13 |
| Colloidal Silicion Dioxide | 1.60 | 1.60 | 0.50 |
| Magnesium Stearate | 1.75 | 1.75 | 0.55 |
| **Total Tablet Weight (Core)** | 320.00 | 320.00 | 100.00 |

The process used to prepare the tablet described above was as follows:
A Prescreen Alendronate Sodium Trihydrate, Lactose Anhydrous, Plasdone S630, and Croscarmellose Sodium NF through a #20 mesh and blend for 10 minutes.
B Prescreen Microcrystalline Cellulose PH 302 and Colloidal Silicone Dioxide through a #20 mesh. Prescreen Cholecalciferol Vitamin D through a #30 mesh and blend for 10 minutes.
C Prescreen Magnesium Stearate Non Bovine through a #60 mesh and blend for 3 minutes.
D Tablet at 15-18Kp.

The dissolution times of resulting tablets were tested and compared to dissolution times for tablets of a commercially available alendronate/Nitamin D tablet (Fosamax D). Dissolution was tested in water using the procedure as set out in USP 23 third supplement.

The results depicted in Figure 2 demonstrate that there was essentially no difference between the Dissolution profiles of the tablets of the present invention and of the commercially available tablets. In the present study, the similarity factor, f₂ was calculated to be 62.3. Dissolution profiles are recognized to be similar if 50<f₂<100.

All publications, patents and patent applications mentioned in this Specification are indicative of the level of skill of those skilled in the art to which this invention pertains and are herein incorporated by reference to the same extent as if each individual publication, patent, or patent applications was specifically and individually indicated to be incorporated by reference.

The invention being thus described, it will be obvious that the same may be varied in many ways. Such vacations are not to be regarded as a departure from the spirit and scope of the invention, and all such modifications as would be obvious to one skilled in the art are intended to be included within the scope of the following claims.

features of the invention:
1. A pharmaceutical composition comprising:
   (a) an active pharmaceutical ingredient (API), pharmaceutically acceptable salts thereof, derivatives or hydrates of the AFI, or mixtures thereof;
   (b) a vitamin D compound; and
   (c) one or more excipients selected from fillers, diluents, binders, lubricants or disintegrants, wherein said composition does not contain a glidant.
2. The pharmaceutical composition according to feature 1, which comprises includes microcrystalline cellulose and lactose.
3. A pharmaceutical composition comprising:
   (a) an active pharmaceutical ingredient (API), pharmaceutically acceptable salts thereof, derivatives or hydrates of the API, or mixtures thereof;
   (b) a vitamin D compound;
   (c) microcrystalline cellulose;
   (d) lactose; and
   (e) one or more excipients selected from fillers, diluents, binders, lubricants disintegrants or glidant, wherein said glidant is present at a concentration of from 0 to 0.5% w/w.
4. The pharmaceutical composition of feature 2 or 3, wherein the lactose is lactose anhydrous and has a density of no less than 0.5 g/ml and the microcrystalline cellulose has a density of no less than 0.4 g/ml.
5. The pharmaceutical composition of any one of features 1-4, wherein the API is a bisphosphonic acid or a pharmaceutically acceptable salt thereof.
6. The pharmaceutical composition of feature 4, wherein the bisphosphonic acid is Etidronate, Clodronate, Tiludronate, Pamidronate, Neridronate, Olpadronate, Alendronate, Ibandronate, Risedronate or Zoledronate.
7. The pharmaceutical composition of feature 5, wherein the API is alendronate monosodium, alendronate monosodium monohydrate, or alendronate monosodium trihydrate.
8. The pharmaceutical composition of any one of features 1-7, wherein the vitamin D compound is cholecalciferol.
9. The pharmaceutical composition according to feature 8, wherein the amount of the vitamin D compound is from about 2,800 IU to about 5600 IU and the amount of API or a pharmaceutically acceptable salt thereof is about 70 mg.
10. The pharmaceutical composition according to feature 9, wherein the amount of the vitamin D compound is about 2800 IU or about 5600 IU.
11. The pharmaceutical composition of feature 9, which comprises plasdone, croscarmellose sodium, magnesium stearate or a combination thereof.
12. The pharmaceutical composition of feature 2 or 3, wherein the lactose is lactose anhydrous, spray dried lactose or spray dried lactose anhydrous.
13. The pharmaceutical composition of feature 2 or 3, wherein the microcrystalline cellulose is Avicel 302.
14. The pharmaceutical composition according to any one of features 1 - 13, which is a solid oral pharmaceutical.
15. The pharmaceutical composition of featurte 13, wherein the solid oral pharmaceutical is a tablet or a capsule.
16. The pharmaceutical composition according to feature to 12, wherein the solid oral pharmaceutical consists of spray dried Lactose Anhydrous, Avicel 302, Atendronate Sodium Trihydrate, Plasdone, Croscarmellose Sodium, Magnesium Stearate and Cholecalciferol Vitamin D.
17. The pharmaceutical composition according to feature 16, wherein the spray dried lactose anhydrous has a density of 0.67 g/ml and the Avicel 302 has a density of 0.45 g/ml.
18. A process for preparing a pharmaceutical composition according to any one of features 1-17, which comprises the steps of:
   (a) blending the active pharmaceutical ingredient (API), pharmaceutically acceptable salts thereof, derivatives or hydrates of the API, or mixtures thereof with a vitamin D compound; and
   (b) adding one or more excipients selected from the group comprising fillers, diluents, binders, lubricants, disintegrants and glidant, wherein said glidant is added so that the final concentration of glidant is from 0 to 0.5% w/w in the final pharmaceutical composition.
19. A method for treating a disorder associated with increased bone resorption comprising administering to a patient a pharmaceutical composition according to any one of features 1-17.
20. The method of feature 19, wherein the disorder is osteoporosis, hypercalcaemia, tumour osteolysis or Paget's disease.
21. The method of feature 19 or 20, wherein the patient has a vitamin D deficiency or insufficiency.
22. A pharmaceutical composition according to any one of features 1 - 17 for treating a disorder associated with increased bone resorption.
23. The pharmaceutical composition according to feature 22, wherein the disorder is osteoporosis, hypercalcaemia, tumour osteolysis or Paget's disease, optionally in combination with a vitamin D deficiency or insufficiency.
24. Use of a pharmacautical composition according to any one of features 1-17 for treating a disorder associated with increased bone resorption.
25. The use according to feature 24, wherein the disorder is osteoporosis, hypercalcaemia, tumour osteolysis or Paget's disease, optionally in combination with a vitamin D deficiency or insufficiency.

## Claims

1. A pharmaceutical composition comprising:
(a) a bisphosphonic acid or a pharmaceutically acceptable salt, derivative or hydrate of the bisphosphonic acid, or mixtures thereof;
(b) cholecalciferol;
(c) plasdone; and
(d) optionally one or more excipients selected from fillers, diluents, binders, lubricants or disintegrants;
wherein said composition does not contain a glidant; and
wherein the amount of the cholecalciferol is from about 2800 IU to about 5600 IU and the amount of the biphosphonic acid or pharmaceutically acceptable salt thereof is about 70 mg.

2. The pharmaceutical composition of claim 1, which comprises microcrystalline cellulose and lactose.

3. A pharmaceutical composition comprising:
(a) a bisphosphonic acid or a pharmaceutically acceptable salt, derivative or hydrate of the bisphosphonic acid, or mixtures thereof;
(b) cholecalciferol;
(c) microcrystalline cellulose;
(d) lactose;
(e) plasdone; and
(f) optionally one or more excipients selected from fillers, diluents, binders, lubricants, disintegrants or glidant;
wherein said glidant is present at a concentration of from 0 to 0.5% w/w; and
wherein the amount of the cholecalciferol is from about 2800 IU to about 5600 IU and the amount of the biphosphonic acid or pharmaceutically acceptable salt thereof is about 70 mg.

4. The pharmaceutical composition of claim 3, wherein said glidant is colloidal silicon dioxide or talc.

5. The pharmaceutical composition of any one of claims 2 - 4, wherein the lactose is lactose anhydrous and has a density of no less than 0.5 g/ml and the microcrystalline cellulose has a density of no less than 0.4 g/ml.

6. The pharmaceutical composition of any one of claims 2 - 5, wherein:
(a) the lactose is lactose anhydrous, spray dried lactose or spray dried lactose anhydrous; and/or
(b) the microcrystalline cellulose is Avicel 302.

7. The pharmaceutical composition of any one of claims 1 - 6, wherein the bisphosphonic acid or pharmaceutically acceptable salt thereof is:
(a) Etidronate, Clodronate, Tiludronate, Pamidronate, Neridronate, Olpadronate, Alendronate, Ibandronate, Risedronate or Zoledronate; or
(b) alendronate monosodium, alendronate monosodium monohydrate, or alendronate monosodium trihydrate.

8. The pharmaceutical composition of any one of claims 1 - 7, wherein the amount of the cholecalciferol is about 2800 IU or about 5600 IU.

9. The pharmaceutical composition of any one of claims 1 - 8, which comprises croscarmellose sodium, magnesium stearate or a combination thereof.

10. The pharmaceutical composition of any one of claims 1 - 9, which is:
(a) a solid oral pharmaceutical; and/or
(b) a tablet or a capsule; and/or
(c) a solid oral pharmaceutical consisting of spray dried Lactose Anhydrous, Avicel 302, Alendronate Sodium Trihydrate, Plasdone, Croscarmellose Sodium, Magnesium Stearate and Cholecalciferol Vitamin D; and/or
(d) a solid oral pharmaceutical consisting of spray dried Lactose Anhydrous, Avicel 302, Alendronate Sodium Trihydrate, Plasdone, Croscarmellose Sodium, Magnesium Stearate and Cholecalciferol Vitamin D, wherein the spray dried Lactose Anhydrous has a density of 0.67 g/ml and the Avicel 302 has a density of 0.45 g/ml.

11. A solid oral pharmaceutical composition consisting of spray dried Lactose Anhydrous, Avicel 302, Alendronate Sodium Trihydrate, Plasdone, Croscarmellose Sodium, Magnesium Stearate and Cholecalciferol Vitamin D.

12. A process for preparing a pharmaceutical composition according to any one of claims 1 - 11, which comprises the steps of:
(a) blending the biphosphonic acid or acceptable salt, derivative or hydrate of the bisphosphonic acid, or mixtures thereof, with cholecalciferol;
(b) adding plasdone; and
(c) optionally adding one or more excipient(s) selected from the group comprising fillers, diluents, binders, lubricants, disintegrants and glidant, wherein said glidant is added so that the final concentration of glidant is from 0 to 0.5% w/w in the final pharmaceutical composition.

13. A pharmaceutical composition of any one of claims 1 - 11 for use as a medicament.

14. A pharmaceutical composition of any one of claims 1 - 11 for use in treating a disorder associated with increased bone resorption, such as a disorder selected from osteoporosis, hypercalcaemia, tumour osteolysis or Paget's disease, optionally in combination with a vitamin D deficiency or insufficiency.

15. Use of a pharmaceutical composition according to any one of claims 1 - 11 for the manufacture of a medicament for treating a disorder associated with increased bone resorption, such as a disorder selected from osteoporosis, hypercalcaemia, tumour osteolysis or Paget's disease, optionally in combination with a vitamin D deficiency or insufficiency.
